# EUROPEAN PATENT APPLICATION

(11) **EP 2 106 781 A1**
(43) Date of publication of application: **07.10.2009**
(21) Application number: 09004601.2
(22) Date of filing: 30.03.2009
(51) Int. Cl.: A61K 6/10, A61K 6/093

(54) **Dental silicone-based fitting check material composition**

(30) Priority: 31.03.2008 JP 2008092351
(71) Applicant: GC Corporation, Itabashi-ku Tokyo 174-8585 (JP)
(72) Inventor:
(74) Representative: Müller-Boré & Partner Patentanwälte

(57) **Abstract**

To provide a dental silicone-based fitting check material composition capable of confirming a precise difference of film thicknesses and having a wide confirmation range of the film thicknesses, the dental silicone-based fitting check material includes 100 weight parts of organopolysiloxane having at least two aliphatic unsaturated hydrocarbons in one molecule, 0.1 to 30 weight parts of organohydrogenpolysiloxane having at least three hydrogen atoms directly bonding to silicon atoms in one molecule, 10 ppm to 1 weight parts of a silicone-soluble platinum compound with respect to the total weight of the above two components, 0.05 to 20 weight parts of powdery palladium, 10 to 800 weight parts of an inorganic filler, and 0.01 to 10 weight parts of a pigment selected from phthalocyanine blue, cobalt blue, and ultramarine.

## Description

The present invention relates to a fitting check material used for confirming a fitting state of a produced dental prosthesis such as a crown, an inlay, or a denture, when the dental prosthesis is produced.

In a dental treatment, a recovery treatment of an oral function lost due to dental caries or extraction of a tooth is carried out by producing a dental prosthesis such as a crown, an inlay, or a denture. For production of a general dental prosthesis, an intraoral model reproducing an intraoral state is produced by taking an impression of an intraoral tissue of teeth or oral mucosa, and pouring gypsum into the impression material. Then, a dental prosthesis is produced by a lost wax method based on the intraoral model. However, in many cases, the produced dental prosthesis has low dimensional accuracy and thus cannot sufficiently fit to an abutment tooth. Further, in recent years, it is widely used for a production method of a dental prosthesis that a metallic or ceramics block is milled by a CAD/CAM system with using the model produced by taking the impression. Although this method can produce a dental prosthesis without casting, the dimensional accuracy of the dental prosthesis is lower than that produced by the lost wax method. Therefore, when the final fitting between the produced dental prosthesis and the abutment tooth is insufficient, it is necessary to slightly polish an inner face or an outer part of the produced dental prosthesis so as to adjust the position of the dental prosthesis to a proper position. At this time, a fitting check material is used for inspecting which portion and how much amount should be polished.

As for a manner of use, the fitting check material is coated on an inner face of a dental prosthesis and removed after the dental prosthesis is attached to an abutment tooth. When a contact failure occurs between the dental prosthesis and the abutment tooth, the inner face of the dental abutment and the abutment tooth are strongly contacted partially, and thus a film of the fitting check material on the inner face of the dental prosthesis is thinned remarkably in comparison with other portions. By polishing the portion where the fitting check material is thinned, the partial fitting failure of the dental prosthesis is improved.

For a conventional fitting check material, Japanese Patent Application Laid-Open No. 5-154168 discloses a dental paste composition, which contains at least one kind of an oil liquid composition selected from a group of silicone oil, liquid paraffin, polybutene, and aliphatic acid, and also contains at least one kind of a metal compound powder selected from a group of a metal oxide, a metal carbonate, and a metal sulfate, which is white and has a water repellent surface. This dental paste composition is for a denture base, has high covering property, and is for inspecting the fitting between a denture base and mucous. Further, Japanese Patent Application Laid-Open No. 11-335224 discloses a denture base fitting check composition consisting of one or two or more kinds of polysiloxanes selected from dimethylpolysiloxane, methylhydrogenpolysiloxane, and methylphenylpolysiloxane, one or two or more kinds of silicates selected from zirconium silicate, calcium silicate, aluminum silicate, magnesium silicate, and zinc silicate, and a surfactant, within predetermined weight ratios.

However, the conventional fitting check material is in white color, and so the check material has the same color as that of a recent white ceramics material produced by CAD/CAM. Therefore, a difference of thicknesses is hardly observed to cause a problem. Further, conversely, when the color tone difference is too large, a precise difference of the thicknesses cannot be precisely observed to cause a problem.

The present invention is directed to provide a dental silicone-based fitting check material composition capable of confirming a precise difference of film thicknesses, even when a dental prosthesis is made of ceramics, metal, or a denture base resin, and applicable to a wide range of film thicknesses.

Present inventors carried out earnest works to solve the aforementioned problems and, as a result, they found out the followings to complete the present invention. The aforementioned problems can be solved when organopolysiloxane having at least two aliphatic unsaturated hydrocarbons in one molecule, organohydrogenpolysiloxane having at least three hydrogen atoms directly bonding to silicon atoms in one molecule, a silicone-soluble platinum compound, powdery palladium, an inorganic filler, and a pigment selected from phthalocyanine blue, cobalt blue, and ultramarine are included.

An aspect of the present invention is a dental silicone-based fitting check material composition, in which 100 weight parts of organopolysiloxane having at least two aliphatic unsaturated hydrocarbons in one molecule, 0.1 to 30 weight parts of organohydrogenpolysiloxane having at least three hydrogen atoms directly bonding to silicon atoms in one molecule, 10 ppm to 1 weight parts of a silicone-solubleplatinumcompoundwithrespecttothe total weight of the above two components, 0.05 to 20 weight parts of powdery palladium, 10 to 800 weight parts of an inorganic filler, and 0.01 to 10 weight parts of a pigment selected from phthalocyanine blue, cobalt blue, and ultramarine are included.

The dental composition according to the present invention is a dental silicone-based fitting check material composition capable of confirming a precise difference of film thicknesses and having a wider visual confirmation range of film thicknesses than that of a conventional fitting check material.

The organopolysiloxane having at least two aliphatic unsaturated hydrocarbons in one molecule used for a dental composition according to the present invention is the same as that used for a general addition type silicone impression material, and is organopolysiloxane having at least two aliphatic unsaturated groups in one molecule. The organopolysiloxane preferably has a linear chain shape, where both terminals of a molecular chain are blocked with a vinylsilyl group. The terminal vinyl group may be plural, and the vinyl groups may be contained in the chain. The organopolysiloxane is the same component as that used for the general addition type silicone impression material.

The organohydrogenpolysiloxane having at least three hydrogen atoms directly bonding to silicon atoms in one molecule needs to have at least three hydrogen atoms directly bonding to silicon atoms in the molecule, and functions as a crosslinking agent. The blending amount of the organohydrogenpolysiloxane is 0.1 to 30 weight parts with respect to 100 weight parts of the organopolysiloxane having at least two aliphatic unsaturated hydrocarbons in one molecule. If the blending amount is less than 0.1 weight parts, the hardness of a set material decreases and the hardening rate also decreases. If the blending amount is more than 50 weight parts, a lot of bubbles due to hydrogen gas are generated in the set material, and thus a correct fitting state cannot be confirmed. The most suitable range of the blending amount is 1 to 20 weight parts.

As for the silicone-soluble platinum compound, a publicly known addition reaction catalyst, such as chloroplatinic acid, alcohol-modified chloroplatinic acid, and a complex of chloroplatinic acid and olefin, can be used. Preferably, a vinylsiloxane complex of chloroplatinic acid is used. The adding amount of the silicone-soluble platinum compound is within the range from 10 ppm to 1 weight part with respect to the total amount of the aforementioned two components. If the adding amount is less than 10 ppm, there occurs problems that the hardening rate is low, and in a case that a minute amount of a material which inhibits a catalytic ability of the platinum compound, is contained, the hardening rate decreases. If the adding amount is more than 1 weight part, the hardening rate is too high and there is economical disadvantage. The silicone-soluble platinum compound of chloroplatinic acid is preferably used after being dissolved with an alcohol-based, ketone-based, ether-based, or hydrocarbon-based solvent, polysiloxane oil or the like.

By using powdery palladium together with a specific pigment mentioned below, the visual fitting confirmation of the fitting check material can be made easy, even when the check material becomes a thin film at a time of a fitting check, to any kinds of materials of the dental prosthesis. As for this powdery palladium, powdery palladium supported by powder such as silica or fine powder of palladium metal itself can be used, but the powdery palladium supported by powder such as silica can be preferably used. The content of the powdery palladium is 0.05 to 20 weight parts with respect to 100 weight parts of the organopolysiloxane having at least two aliphatic unsaturated hydrocarbons in one molecule. If the content is less than 0.05 weight parts, the fitting cannot be fully confirmed when the check material becomes a thin film. If the content is more than 20 weight parts, the color tone of the set material is too deep, the difference of partial fitting state cannot be visually determined. The preferable content is 0.1 to 5% by weight.

The inorganic filler is to give a proper hardness and operativity to a composition. As for the inorganic filler, quartz, cristobalite, diatomite, fused quartz, glass fiber, titanium dioxide, fumed silica, and the like can be used. The blending amount of the inorganic filler is 10 to 800 weight parts with respect to 100 weight parts of the organopolysiloxane having at least two aliphatic unsaturated hydrocarbons in one molecule. If the blending amount is less than 10 weight parts, the set material is made weak. If the blending amount is more than 800 weight parts, viscosity of the composition is too high so that the film is excessively thickened. Thus, the correct fitting check cannot be carried out.

When the specific pigment selected from phthalocyanine blue, cobalt blue, and ultramarine is used with the powdery palladium, a wide range of thickness difference can be visually confirmed easily. Particularly, the fitting of a dental prosthesis made of ceramics can be visually confirmed easily. The blending amount of the specific pigment is 0.01 to 10 weight parts with respect to 100 weight parts of the organopolysiloxane having at least two aliphatic unsaturated hydrocarbons in one molecule. If the blending amount is less than 0.01 weight parts, the thickness difference of the film cannot be visually confirmed. If the blending amount is more than 10 weight parts, only a remarkably thin portion of the film can be confirmed. The preferable blending amount is 0.05 to 5 weight parts.

The silicone-based fitting check material composition according to the present invention can contain various kinds of silicone resins, a non-reactive silicone oil, polyether containing aliphatic unsaturated hydrocarbon, surfactant, and the like within a range not losing the properties of the composition.

### [Example]

The present invention will be described in detail below with examples, but is not limited to these examples.

### <Example 1>

A base paste and a catalyst paste having following compositions were produced.

### (Base paste)

Dimethylpolysiloxane in which both terminals of the molecular chain are blocked with a methylvinylsiloxy group: 100 weight parts
Linear chain methylhydrogenpolysiloxane containing a methylhydrogensiloxane unit of 45% by mol: 6 weight parts
Phthalocyanine blue: 0.5 weight parts
Quarts (an average particle diameter of 7µm): 30 weight parts

### (Catalyst paste)

Dimethylpolysiloxane in which both terminals of the molecular chain are blocked with a dimethylvinylsiloxy group: 100 weight parts
Silicone oil solution containing 1.3 divinyltetramethyldisiloxane / platinum complex of 0.8% by weight: 1 weight part
Phthalocyanine blue: 0.5 weight parts
Quarts (an average particle diameter of 7µm): 25 weight parts
Quartz powder having an average particle diameter of 4µm and a support ratio of palladium of 5% by weight: 0.5 weight parts

### <Visual confirming of thickness difference of films>

A set material having a thickness of 10µm was produced by weighing 5g of the base paste and the catalyst paste respectively, kneading those, and pressing the mixture by glass plates. Similarly, films of the set material, the thicknesses of which vary every 10µm in a range from 10µm to 100µm, were produced. Then, each film was put on a plate made of a dental metal (the product name: CASTWELL, produced by GC Corporation) or a dental ceramics body (the product name: GN-CERAM, produced by GC Corporation), and it was confirmed whether the difference of each film of 10µm to 100µm was clearly determined visually. These results were shown in Table 1.

### <Confirmation of fitting state>

After a gypsum model was produced by taking an impression of a molar part resin model formed to have an abutment tooth, a crown was produced by using the aforementioned CASTWELL or GN-CERAM. After confirming that the crown was not sufficiently fitted to the molar part resin model due to its bad fitting, the fitting check was carried out by using the fitting check material composition. Then, a portion, which is visually determined to be unfitted, was polished and re-attached to the molar part resin model. It was confirmed whether the problems, such as floating of the crown and the like, exist at a margin part of the crown after the adjustment of the fitting. These results were shown in Table 1.

### <Example 2>

Abase paste and a catalyst paste having following compositions were produced.

### (Base paste)

Dimethylpolysiloxane in which both terminals of the molecular chain are blocked with a methylvinylsiloxy group: 100 weight parts
Linear chain methylhydrogenpolysiloxane containing a methylhydrogensiloxane unit of 45% by mol: 1 weight part
Cobalt blue: 5 weight parts
Quarts (an average particle diameter of 7µm): 100 weight parts

### (Catalyst paste)

Dimethylpolysiloxane in which both terminals of the molecular chain are blocked with a dimethylvinylsiloxy group: 100 weight parts
Silicone oil solution containing 1,3 divinyltetramethyldisiloxane / platinum complex of 0.7% by weight: 1 weight part
Cobalt blue: 8 weight parts
Quarts (an average particle diameter of 7µm: 100 weight parts
Quartz powder having an average particle diameter of 4µm and a support ratio of palladium of 5% by weight: 0.1 weight parts

The composition was subjected to the same test as that of Example 1.

### <Example 3>

Abase paste and a catalyst paste having following compositions were produced.

### (Base paste)

Dimethylpolysiloxane in which both terminals of the molecular chain are blocked with a methylvinylsiloxy group: 100 weight parts
Linear chain methylhydrogenpolysiloxane containing a methylhydrogensiloxane unit of 10% by mol: 10 weight parts
Phthalocyanine blue: 1 weight parts
Quarts (an average particle diameter of 7µm): 200 weight parts
Silicone resin: 20 weight parts
Polypropyleneglycoldiallyl ether in which both terminals of the molecular chain are blocked with a vinyl group: 5 weight parts

### (Catalyst paste)

Dimethylpolysiloxane in which both terminals of the molecular chain are blocked with a dimethylvinylsiloxy group: 100 weight parts
Silicone oil solution containing 1.3 divinyltetramethyldisiloxane / platinum complex of 0.7% by weight: 2 weight parts
Phthalocyanine blue: 1 weight parts
Quarts (an average particle diameter of 7µm): 200 weight parts
Silicone resin: 20 weight parts
Quartz powder having an average particle diameter of 4µm and a support ratio of palladium of 5% by weight: 5 weight parts

The above pastes were subjected to the same test as that of Example 1.

### <Comparative example 1>

The same test as that of the examples was carried out by using a commercial fitting check material (the product name: FIT CHECKER, produced by GC Corporation) These results were shown in Table 1.

### <Comparative example 2>

A composition was produced by removing the powdery palladium and the pigment from the composition of Example 1, and was subjected to the same test as that of the examples. These results were shown in Table 1.

### (Base paste)

Dimethylpolysiloxane in which both terminals of the molecular chain are blocked with a methylvinylsiloxy group: 100 weight parts
Linear chain methylhydrogenpolysiloxane containing a methylhydrogensiloxane unit of 45% by mol: 6 weight parts
Quarts (an average particle diameter of 7µm: 30 weight parts

### (Catalyst paste)

Dimethylpolysiloxane in which both terminals of the molecular chain are blocked with a dimethylvinylsiloxy group: 100 weight parts
Silicone oil solution containing 1,3 divinyltetramethyldisiloxane / platinum complex of 0.8% by weight: 1 weight part
Quarts (an average particle diameter of 7µm: 25 weight parts

**<Table 1>**

| | Examples | | | Comparative examples | |
|---|---|---|---|---|---|
| | 1 | 2 | 3 | 1 | 2 |
| Visual confirmation of thickness of thickness | Differences of thicknesses from 10 to 100µm were clearly confirmed. | Differences of thicknesses from 10 to 100µm were clearly confirmed. | Differences of thicknesses from 10 to 100µm were clearly confirmed. | Differences of thicknesses of 50µm or more were not clearly confirmed. Particularly, differences of any thicknesses were not confirmed on a ceramics plate. | Differences of thicknesses from 10 to 60µm were not clearly confirmed. |
| Confirmation of fitting state of fitting state | Floating at a margin part was part was not observed | Floating at a margin part was part was not observed | Floating at a margin part was part was not observed | A margin part was floated a was floated a little. | A margin part was floated a was floated a little. |

Clearly from Table 1, as for the dental silicone-based fitting check material compositions of the examples, the difference of the film thicknesses could be clearly confirmed visually, and the fitting property could be confirmed with high accuracy regardless of a kind of the material of the dental prosthesis, such as a metal or a ceramics. Particularly, it was confirmed that the commercial fitting check material of Comparative example 1 could hardly be used for a fitting inspection of a ceramics. Therefore, it was clearly confirmed that the fitting check material of the present invention was a fitting check material in which conventional problems were greatly improved.

## Claims

1. A dental silicone-based fitting check material, wherein 100 weight parts of organopolysiloxane having at least two aliphatic unsaturated hydrocarbons in one molecule, 0.1 to 30 weight parts of organohydrogenpolysiloxane having at least three hydrogen atoms directly bonding to silicon atoms in one molecule, 10 ppm to 1 weight parts of a silicone-solubleplatinumcompoundwithrespecttothe total weight of the above two components, 0.05 to 20 weight parts of powdery palladium, 10 to 800 weight parts of an inorganic filler, and 0.01 to 10 weight parts of a pigment selected from phthalocyanine blue, cobalt blue, and ultramarine are included.
